# DEMANDE DE BREVET EUROPEEN

(11) **EP 0 613 898 A1**
(43) Date de publication de la demande: **07.09.1994**
(21) Numéro de dépôt: 94400448.0
(22) Date de dépôt: 03.03.1994
(51) Int. Cl.: C07D 417/06, C07D 413/06, C07D 277/68, C07D 263/58, A61K 31/445, A61K 31/495

(54) **Nouveaux composés (aryl(alkyl)carbonyl)-hétérocycliques leurs procédés de préparation et les compositions qui les contiennent**

(30) Priorité: 05.03.1993 FR 9302528
(71) Demandeur: ADIR ET COMPAGNIE, F-92415 Courbevoie Cédex (FR)
(72) Inventeur: Diouf, Ousmane, F-59650 Villeneuve d'Ascq (FR); Lesieur, Daniel, F-59147 Gondecourt (FR); Depreux, Patrick, F-59280 Armentieres (FR); Guardiola-Lemaitre, Béatrice, F-92210 Saint-Cloud (FR); Caignard, Daniel-Henri, F-75015 Paris (FR); Renard, Pierre, F-78000 Versailles (FR); Adam, Gérard, F-78600 Le Mesnil le Roi (FR)

(57) **Abrégé**

L'invention concerne les composés de formule (I): 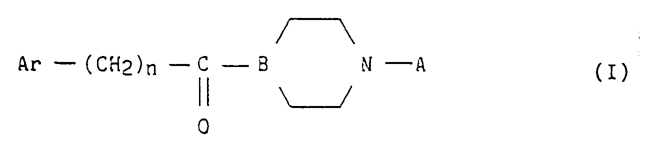
dans laquelle
Ar: représente un groupement phényle ou naphtyle, Ar étant non substitué ou substiuté par un ou plusieurs radicaux choisis parmi halogène, alkyle inférieur, alkoxy inférieur et trifluorométhyle,
n: représente 0 ou un nombre entier de 1 à 4,
B: représente un groupement 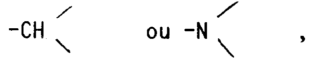 et
A: représente un groupement de formule (A1) ou A2): 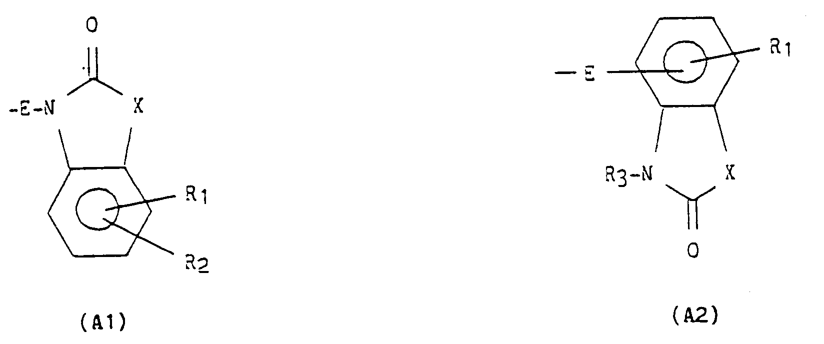 dans lesquelles:
E: représente une chaîne alkylène linéaire ou ramifiée de 1 à 6 atomes de carbone;
R₁: représente un radical choisi parmi hydrogène, hydroxy, alkyle inférieur et alkoxy inférieur,
R₂: représente un radical choisi parmi hydrogène, alkyle inférieur, et 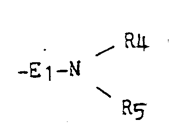 où E₁ a la même définition que E tel que décrit précédemment et où R₄ et R₅ sont choisis indépendamment l'un de l'autre parmi hydrogène et alkyle inférieur, ou forment ensemble, avec l'atome d'azote qui les porte, un hétérocycle choisi parmi pyrrolidine, pipéridine, pipéridine substituée, morpholine, pipérazine, et pipérazine substituée,
R₃: représente un radical choisi parmi hydrogène et alkyle inférieur, et
X: représente un atome de soufre ou d'oxygène,

leurs isomères optiques,
et leurs sels d'addition à une base ou a un acide pharmaceutiquement acceptable, et leur utilisation comme medicaments.

## Description

La présente invention concerne de nouveaux composés (aryl(alkyl)carbonyl)hétérocycliques, leurs procédés de préparation et les compositions pharmaceutiques qui les contiennent.

Il est connu que les récepteurs sérotoninergiques 5-HT2 sont associés à une amélioration des états schizophréniques. Ils ont également montré des effets bénéfiques dans l'anxiété et la dépression.

Des composés possédant une bonne affinité pour ces récepteurs 5-HT₂ seraient donc utiles en clinique pour le traitement de ces pathologies.

La demanderesse a découvert de nouveaux dérivés (aryl(alkyl)carbonyl) hétérocycliques possédant une forte affinité pour les récepteurs 5HT₂, certains de ces dérivés possédant également de façon surprenante une activité analgésique.

Il est connu de l'état de l'art (demande de brevet EP 506539) des composés hétérocycliques, décrits comme possédant une affinité pour les récepteurs mélatoninergiques mais ces composés ne présentent aucune affinité comparable pour les récepteurs 5HT-2, ni ne présentent d'activité analgésique.

Plus particulièrement, l'invention concerne les composés de formule (I) : 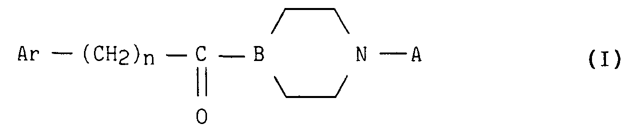
dans laquelle :
- Ar :: représente un groupement phényle ou naphtyle, Ar étant non substitué ou substitué par un ou plusieurs radicaux choisis parmi halogène, alkyle inférieur, alkoxy inférieur et trifluorométhyle,
- n :: représente 0 ou un nombre entier de 1 à 4,
- B :: représente un groupement -CH〈 ou -N〈 , et
- A :: représente un groupement de formule (A1) ou (A2) :

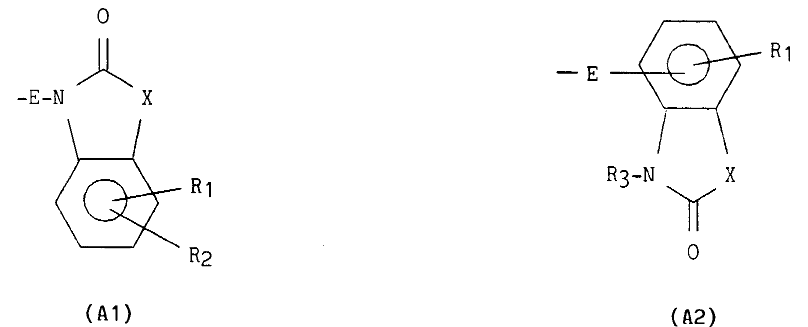

dans lesquelles :
- E :: représente une chaîne alkylène linéaire ou ramifiée de 1 à 6 atomes de carbone,
- R₁ :: représente un radical choisi parmi hydrogène, hydroxy, alkyle inférieur et alkoxy inférieur,
- R₂ :: représente un radical choisi parmi hydrogène, alkyle inférieur, et 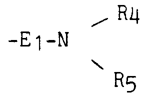 où E1 a la même définition que E tel que décrit précédemment et où R₄ et R₅ sont choisis indépendamment l'un de l'autre parmi hydrogène et alkyle inférieur, ou forment ensemble, avec l'atome d'azote qui les porte, un hétérocycle choisi parmi pyrrolidine, pipéridine, pipéridine substituée, morpholine, pipérazine, et pipérazine substituée,
- R₃ :: représente un radical choisi parmi hydrogène et alkyle inférieur, et
- X :: représente un atome de soufre ou d'oxygène,

leurs isomères optiques,
et leurs sels d'addition à une base ou à un acide pharmaceutiquement acceptable,
étant entendu que, sauf précision contraire, les termes "alkyle inférieur" et "alcoxy inférieur" désignent des groupements linéaires ou ramifiés de 1 à 6 atomes de carbone, et le terme "substitué" affecté aux hétérocycles "pipéridine" et "pipérazine" signifie que ces hétérocycles peuvent être substitués en position 4 par un radical choisi parmi alkyle inférieur, aryle et arylalkyle inférieur ; le terme "aryle" désignant un groupement phényle, naphtyle ou pyridinyle et pouvant lui-même être non substitué ou substitué par un ou plusieurs radicaux choisis parmi halogène, alkyle inférieur, hydroxyle, alkoxy inférieur et trifluorométhyle.

Parmi les acides pharmaceutiquement acceptables que l'on peut utiliser pour former un sel d'addition avec les composés de l'invention, on peut citer, à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Parmi les bases pharmaceutiquement acceptables que l'on peut utiliser pour salifier les composés utilisés selon l'invention, on peut citer, à titre d'exemples et de façon non limitative la soude, la potasse, la triéthylamine, la diéthylamine, l'éthanolamine, l'arginine, la lysine, et la diéthanolamine.

L'invention s'étend au procédé de préparation des composés de formule (I) caractérisé en ce que l'on fait réagir un composé de formule (II) : 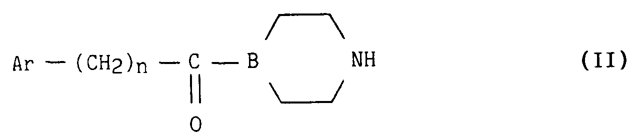
dans laquelle Ar, n et B sont tels que définis dans la formule (I),
avec un composé de formule (III/A1) : 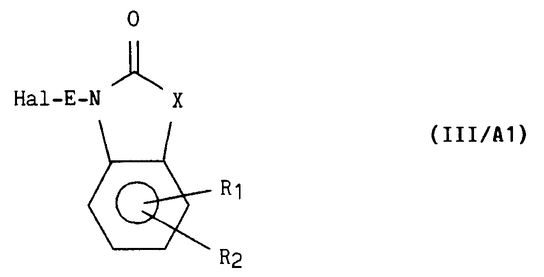
ou de formule (III/A2) : 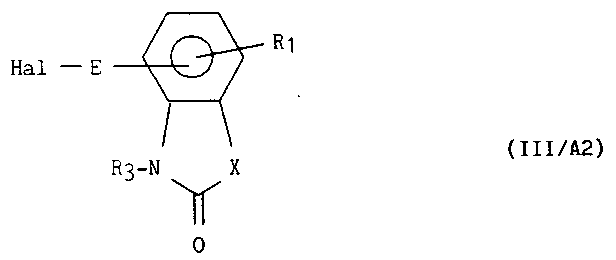
dans lesquelles E, X, R₁, R₂, et R₃ sont tels que définis dans la formule (I) et Hal représente un atome d'halogène, pour obtenir le composé de formule (I) correspondant,
les composés de formule (I) étant le cas échéant :
- purifiés suivant une ou plusieurs méthodes de purification choisies parmi la cristallisation, la chromatographie sur colonne de silice, l'extraction, la filtration, et le passage sur charbon ou résine,
- séparés, sous forme pure ou sous forme de mélange, en leurs isomères optiques,
- et salifiés par un acide ou une base pharmaceutiquement acceptable.

L'invention s'étend également au procédé d'obtention des composés de formule (I/a) : 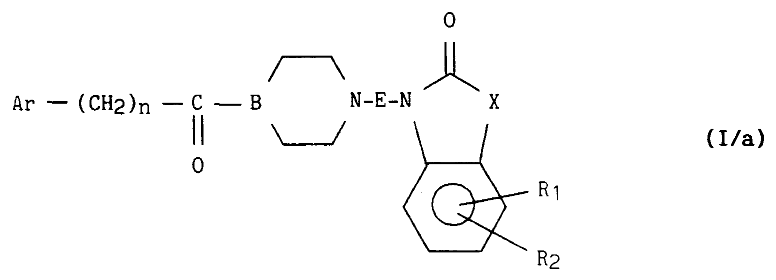
dans laquelle Ar, n, B, E, X, R₁ et R₂ sont tels que définis dans la formule (I),
caractérisé en ce que l'on fait réagir un composé de formule (IV) : 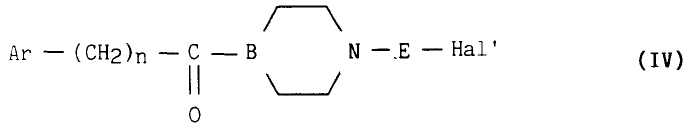
dans laquelle Ar, n, B et E sont tels que définis précédemment et Hal' représente un atome d'halogène, avec un composé de formule (V) : 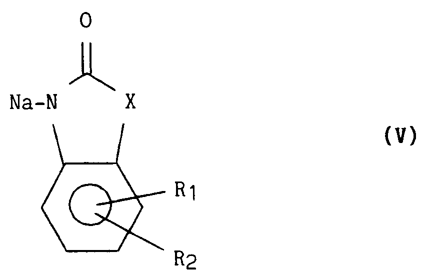
dans laquelle X, R₁ et R₂ sont tels que décrits précédemment, afin d'obtenir les composés de formule (I/a) correspondants,
les composés de formule (I/a) étant le cas échéant :
- purifiés suivant une ou plusieurs méthodes de purification choisies parmi la cristallisation, la chromatographie sur colonne de silice, l'extraction, la filtration, et le passage sur charbon ou résine,
- séparés, sous forme pure ou sous forme de mélange, en leurs isomères optiques,
- et salifiés par un acide ou une base pharmaceutiquement acceptable.

Les matières premières utilisées dans les procédés précédemment décrits sont soit commerciales, soit aisément accessibles à l'homme du métier selon les procédés connus dans la littérature ou proposés lors des exemples de préparations décrits ci-après.

Les composés de formule (II) sont par exemple aisément accessibles à l'homme de l'art par réaction d'un composé de formule (II/a) : 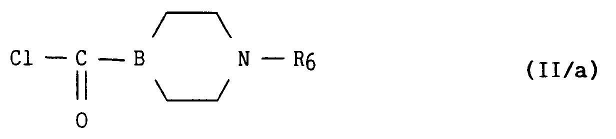
dans laquelle B est tel que décrit dans la formule (I) et R₆ représente un atome d'hydrogène ou un groupement protecteur de l'azote,
avec un composé de formule (II/b) :

Ar (II/b)

dans laquelle Ar est tel que défini dans la formule (I),
ou avec un composé de formule (II/c) :

Ar - (CH₂)ₙ - MgBr (II/c)

dans laquelle Ar et n sont tels que définis dans la formule (I),
pour obtenir, après élimination du groupement protecteur R₆ le cas échéant, les composés de formule (II) correspondants.

Les composés de formule (IV) sont également aisément obtenus par réaction d'un composé de formule (II) : 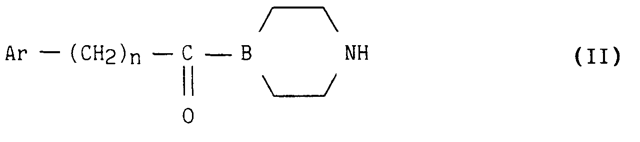
dans laquelle Ar, n et B sont tels que définis dans la formule (I),
avec un alcool de formule (VI) :

Hal'-E-OH (VI)

dans laquelle E est tel que défini dans la formule (I) et Hal' représente un atome d'halogène, afin d'obtenir le composé de formule (IV) correspondant.

Les composés de formule (III/A1) (III/A2) sont aisément accessibles à l'homme du métier selon les procédés analogues à ceux décrits dans la demande EP 506539.

La demanderesse a découvert que les composés de l'invention possédaient une remarquable affinité pour les récepteurs sérotoninergiques 5-HT₂.

Cette capacité de liaison très importante est mise en évidence dans l'exemple A du présent document (Exemple A : Mesure de l'affinité pour les récepteurs sérotoninergiques).

Cette haute affinité pour les récepteurs 5HT-2 présentée par les composés de l'invention s'avère surprenante puisque les composés de l'art antérieur mentionnés dans la demande EP 506539 ne présentent aucunement une telle affinité pour ces récepteurs.

L'activité antipsychotique des composés de l'invention a été montrée sur le test de l'antagonisme de l'hyperactivité induite par l'amphétamine (Exemple B de la présente demande : Etude de l'antagonisme de l'hyperactivité induite par l'amphétamine).

Les composés de l'invention présentent également une activité anxiolytique (Exemple C : Test des cages claires-obscures).

L'activité surprenante également des composés de l'invention comme analgésique est montrée dans l'exemple D (test de la plaque chauffante).

De par leur mode d'action, les composés de l'invention sont donc de nouveaux candidats pour le traitement et la prévention des pathologies nécessitant des agents psychotropiques, et le traitement des états impliquant la douleur.

Les composés de l'invention sont plus particulièrement utiles dans le traitement et la prévention de l'anxiété, de la dépression et des syndrômes dépressifs, des états psychotiques, de la maladie de Parkinson et le traitement de la douleur.

La présente invention a également pour objet les compositions pharmaceutiques contenant comme principe actif un ou plusieurs composés de formule (I), ou leurs sels d'addition à un acide ou à une base pharmaceutiquement acceptable, en combinaison avec un ou plusieurs excipients, véhicules ou diluants pharmaceutiquement acceptables.

Parmi les compositions pharmaceutiques selon l'invention, on pourra citer, à titre d'exemples et de façon non limitative, celles qui conviennent à l'administration orale, parentérale, nasale, rectale, perlinguale, oculaire ou pulmonaire et notamment les préparations injectables, les aérosols, les gouttes oculaires ou nasales, les comprimés simples, pelliculés ou dragéifiés, les gélules, les capsules, les suppositoires, les crèmes, pommades, et les gels dermiques.

La posologie utile varie selon l'âge et le poids du patient, la voie d'administration, la nature de l'affection et des traitememnts éventuellement associés et s'échelonne entre 0,05 mg et 50 mg par 24 heures en 1 à 2 prises.

### EXEMPLE 1 :

**3-{2-[4-(4-FLUOROBENZOYL)PIPERIDINO]ETHYL}BENZOTHIAZOLIN-2-ONE**

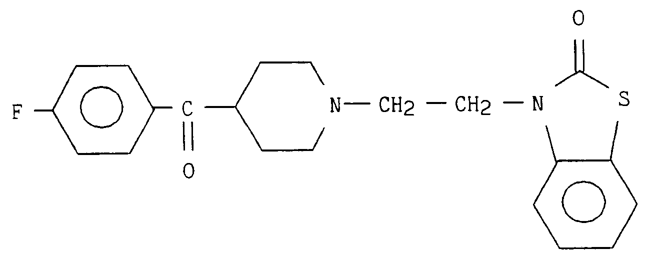

### STADE A :

**1-(2-CHLOROETHYL)-4-(4-FLUOROBENZOYL)PIPERIDINE**
- acide 1-acétyl pipéridinecarboxylique-4
   L'acide 1-acétyl pipéridinecarboxylique-4 est préparé en portant à reflux pendant 2 heures une solution d'acide pipéridinecarboxylique-4 dans de l'anhydride acétique qui est ensuite mis sous agitation pendant 16 heures à température ambiante. La solution est ensuite concentrée et le résidu est trituré dans l'éther. Le composé solide est récupéré par filtration
   Solvant de recristallisation : isopropanol-éther isopropylique
   Point de fusion : 180-182°C
- 1-acétyl 4-(4-fluorobenzoyl)pipéridine
   L'acide 1-acétyl pipéridinecarboxylique-4 est versé dans une solution de chlorure de thionyle. Le chlorure d'acyle formé précipite en solution. Après plusieurs lavages à l'éther de pétrole, le solide est séché. Le spectre infrarouge montre une complète conversion de l'acide en chlorure d'acyle. Le chlorure d'acyle est ajouté lentement à du chlorure d'aluminium en solution dans le fluorobenzène, sous agitation. Le mélange est ensuite porté à reflux pendant 1 heure. Le mélange est versé sur glace et les 2 phases résultantes sont séparées. La phase aqueuse est extraite 2 fois avec du chloroforme et les extraits sont ajoutés au fluorobenzène séparé précédemment. La solution organique est séchée (Na₂SO₄) et filtrée. Le filtrat est concentré sous pression réduite et on obtient un solide blanc cristallin.
   Point de fusion : 75-78°C
- 4-(4-fluorobenzoyl)pipéridine
   Une solution de 1-acétyl 4-(4-fluorobenzoyl)pipéridine dans l'acide chlorhydrique 6N est portée à reflux pendant 2 heures. La solution est refroidie puis extraite 2 fois avec de l'éther. La solution aqueuse est rendue basique (NaOH) puis extraite avec du benzène. Les extraits sont séchés (Na₂SO₄) et filtrés. Le filtrat est concentré sous pression réduite et l'huile résiduelle est convertie en sel de l'acide chlorhydrique.
   Point de fusion (chlorhydrate) : 222-224°C
- 1-(2-chloroéthyl)-4-(4-fluorobenzoyl)pipéridine
   Dans un ballon de 250 cm³ muni d'un réfrigérant à eau, introduire 0,01 mole du chlorhydrate de la 4-(4-fluorobenzoyl)pipéridine dans une solution éthanolique d'hydroxyde de sodium. Porter le mélange réactionnel à reflux et ajouter 0,012 mole du 2-bromoéthanol. Maintenir le reflux pendant deux heures, laisser refroidir, essorer le précipité minéral formé et évaporer l'alcool sous pression réduite.

Reprendre le résidu par 75 cm³ de chloroforme anhydre, refroidir dans un bain de glace, ajouter 0,04 mole de chlorure de thionyle, adapter un réfrigérant à eau et poursuivre la réaction pendant quatre heures à reflux du solvant. Laisser refroidir, évaporer le chloroforme puis, reprendre le résidu par l'alcool absolu et porter à ébullition pour éliminer le chlorure de thionyle.

Evaporer l'alcool absolu et ajouter au résidu une solution acétonique anhydre saturée d'acide chlorhydrique gazeux. Essorer le précipité obtenu puis le recristalliser.
Point de fusion (chlorhydrate) : 110-112°C
Rendement : 65%
Solvant de recristallisation : acétone anhydre

| Analyse élémentaire : | | | |
|---|---|---|---|
| Calculée : | C% 52,27 | H% 6,14 | N% 4,38 |
| Trouvée : | C% 52,68 | H% 6,18 | N% 4,41 |

| Spectrométrie dans l'Infrarouge : | |
|---|---|
| 3000-2600 cm⁻¹ | ν CH (alkyles) |
| 1670 cm⁻¹ | ν CO |
| 1610-1580 cm⁻¹ | ν C=C (aromatiques) |

### STADE B :

**3-{2-[4-(4-FLUOROBENZOYL)PIPERIDINO]ETHYL}BENZOTHIAZOLIN-2-ONE**

Dans une fiole à col rodé de 250 cm³ munie d'un réfrigérant à eau, solubiliser 0,01 mole de benzothiazolin-2-one dans le diméthylformamide. Ajouter 0,06 mole de carbonate de potassium et porter à reflux. Laisser sous agitation pendant trente minutes et ajouter 0,012 mole du chlorhydrate de la 1-(2-chloroéthyl)-4-(4-fluorobenzoyl)pipéridine préalablement solubilisée dans le diméthylformamide. Continuer l'agitation pendant une heure, laisser refroidir, essorer l'insoluble minéral et verser le filtrat sur de la glace pilée.
Essorer le précipité obtenu, le sécher, le dissoudre dans l'acétone anhydre puis, faire précipiter le chlorhydrate en barbotant un courant d'acide chlorhydrique gazeux sec. Essorer le produit obtenu, le sécher puis le recristalliser.
Point de fusion (chlorhydrate) : 254-256°C
Rendement : 67%
Solvant de recristallisation : acétone anhydre

| Dosage d'azote basique : pour un azote basique | |
|---|---|
| Pourcentage d'azote basique théorique : | 3,33% |
| Pourcentage d'azote basique trouvé : | 3,61% |

| Analyse élémentaire : (composé du titre +7/2 H₂O) | | | |
|---|---|---|---|
| Calculée : | C% 52,18 | H% 5,16 | N% 5,95 |
| Trouvée : | C% 52,11 | H% 5,55 | N% 5,78 |

| Spectrométrie dans l'Infrarouge : | |
|---|---|
| 3100-2800 cm⁻¹ | ν CH (alkyles) |
| 2700-2300 cm⁻¹ | ν NH⁺ |
| 1610 cm⁻¹ | ν CO (NCOS + cétones) |
| 1580 cm⁻¹ | ν C=C |

### EXEMPLE 2 :

**3-{2-[4-(4-FLUOROBENZOYL)PIPERIDINO]ETHYL}-6-{4-[4-(3-TRIFLUOROMETHYL****PHENYL)PIPERAZINYL]BUTYL} BENZOTHIAZOLIN-2-ONE**

En procédant comme dans l'exemple 1, mais en remplacant la benzothiazolin-2-one par le dichlorhydrate de la 6-{4-[4-(3-trifluorométhylphényl)piperazinyl]butyl} benzothiazolin-2-one, on obtient le composé du titre.
Point de fusion (trichlorhydrate) : 250-252°C
Rendement : 37%
Solvant de recristallisation : alcool absolu

| Dosage d'azote basique : pour trois azotes basiques | |
|---|---|
| Pourcentage d'azote basique théorique : | 5,39% |
| Pourcentage d'azote basique trouvé : | 5,31% |

| Spectrométrie dans l'Infrarouge : | |
|---|---|
| 3080-2840 cm⁻¹ | ν CH (alkyles) |
| 2500-2000 cm⁻¹ | ν NH⁺ |
| 1670 cm⁻¹ | ν CO (NCOS + cétones) |
| 1580 cm⁻¹ | ν C=C (aromatiques) |

### EXEMPLE 3 :

**3-{2-[4-(4-FLUOROBENZOYL)PIPERIDINO]ETHYL}-6**-{**4-[4-((2-METHOXYPHENYL)** **PIPERAZINYL]BUTYL} BENZOTHIAZOLIN-2-ONE**

En procédant comme dans l'exemple 1, mais en remplacant la benzothiazolin-2-one par le dichlorhydrate de la 6-{4-[4-(2-méthoxyphényl)piperazinyl] butyl} benzothiazolin-2-one, on obtient le composé du titre.
Point de fusion (trichlorhydrate) : 252-254°C
Rendement : 30%
Solvant de recristallisation : alcool absolu

| Dosage d'azote basique : pour trois azotes basiques | |
|---|---|
| Pourcentage d'azote basique théorique : | 5,67% |
| Pourcentage d'azote basique trouvé : | 5,86% |

| Analyse élémentaire : (composé du titre +3 H₂O) | | | |
|---|---|---|---|
| Calculée : | C% 51,60 | H% 5,15 | N% 6,97 |
| Trouvée : | C% 51,93 | H% 5,37 | N% 6,73 |

| Spectrométrie dans l'Infrarouge : | |
|---|---|
| 3100-2800 cm⁻¹ | ν CH (alkyles) |
| 2700-2300 cm⁻¹ | ν NH⁺ |
| 1670 cm⁻¹ | ν CO (NCOS + cétone) |
| 1590 cm⁻¹ | ν C=C (aromatiques) |
| 1020 cm⁻¹ | ν OCH₃ |

### EXEMPLE 4 :

**3-{2-[4-(4-FLUOROBENZOYL)PIPERIDINO]ETHYL}-6-(4-MORPHOLINOBUTYL)** **BENZOTHIAZOLIN-2-ONE**

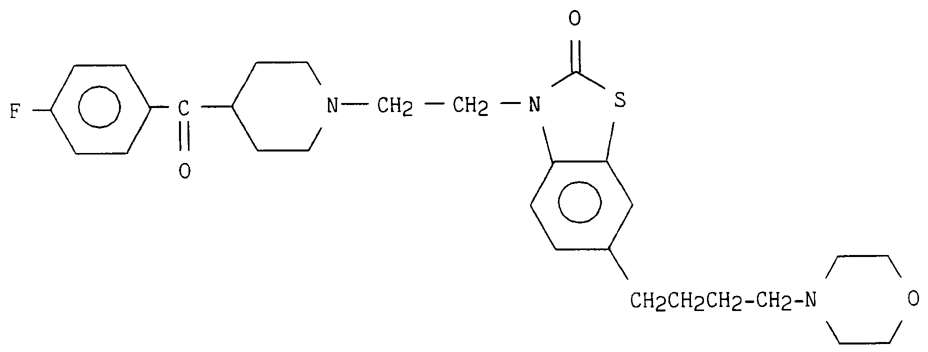

En procédant comme dans l'exemple 1, mais en remplacant la benzothiazolin-2-one par la dichlorhydrate de la 6-(morpholinobutyl)benzothiazolinon-2-one, on obtient le composé du titre.
Point de fusion (dichlorhydrate) : 258-260°C

### EXEMPLES 5 A 22 :

En procédant comme dans l'exemple 1, mais en remplaçant au stade B la benzothiazolin-2-one par :
- la 6-(2-morpholinoéthyl)benzothiazolin-2-one, on obtient :

### EXEMPLE 5 :

**LA 3-{2-[4-(4-FLUOROBENZOYL)PIPERIDINO]ETHYL}-6-(2-MORPHOLINOETHYL)BENZO-** **THIAZOLIN-2-ONE**
- la 6-[2-(4-phénylpipérazinyl)éthyl]benzothiazolin-2-one, on obtient :

### EXEMPLE 6 :

**LA 3-{2-[4-(4-FLUOROBENZOYL)PIPERIDINO]ETHYL}-6-[2-(4-PHENYLPIPERAZINYL)** **ETHYL] BENZOTHIAZOLIN-2-ONE**
- la 6-{2-[4-(3-trifluorométhylphényl)pipérazinyl]éthyl}benzothiazolin-2-one, on obtient :

### EXEMPLE 7 :

**LA** **3-{2-[4-(4-FLUOROBENZOYL)PIPERIDINO]ETHYL}-6-{2-[4-(3-TRIFLUOROMETHYL-****PHENYL)PIPERAZINYL]ETHYL}BENZOTHIAZOLIN-2-ONE**
- la 6-(N,N-dipropylaminoéthyl)benzothiazolin-2-one, on obtient :

### EXEMPLE 8 :

**LA 3-{2-[4-(4-FLUOROBENZOYL)PIPERIDINO]ETHYL}-6-(N,N-DIPROPYLAMINOETHYL)** **BENZOTHIAZOLIN-2-ONE**
- la 6-{2-[4-(2,3,4-triméthoxyphénylméthyl)pipérazinyl]éthyl} benzothiazolin-2-one, on obtient :

### EXEMPLE 9 :

**LA** **3-{2-[4-(4-FLUOROBENZOYL)PIPERIDINO]ETHYL}-6-{2-[4-(2,3,4-TRIMETHOXY-****PHENYLMETHYL) PIPERAZINYL]ETHYL} BENZOTHIAZOLIN-2-ONE**
- la 6-méthoxybenzothiazolin-2-one, on obtient :

### EXEMPLE 10 :

**LA** **3-{2-[4-(4-FLUOROBENZOYL)PIPERIDINO]ETHYL}-6-METHOXYBENZOTHIAZOLIN-2-****ONE**
- la 6-hydroxybenzothiazolin-2-one, on obtient :

### EXEMPLE 11 :

**LA** **3-{2-[4-(4-FLUOROBENZOYL)PIPERIDINO]ETHYL}-6-HYDROXYBENZOTHIAZOLIN-2-****ONE**
- la 5-méthoxybenzothiazolin-2-one, on obtient :

### EXEMPLE 12 :

**LA** **3-{2-[4-(4-FLUOROBENZOYL)PIPERIDINO]ETHYL}-5-METHOXYBENZOTHIAZOLIN-2-****ONE**
Point de fusion : 248-250°C
- la 6-(N,N-dipropylaminoéthyl)-5-méthoxy-benzothiazolin-2-one, on obtient :

### EXEMPLE 13 :

**LA** **3-{2-[4-(4-FLUOROBENZOYL)PIPERIDINO]ETHYL}-6-(N,N-DIPROPYLAMINOETHYL)-****5-METHOXYBENZOTHIAZOLIN-2-ONE**
- la 6-[2-(4-pyridin-3-yl-pipérazinyl)éthyl]benzothiazolin-2-one, on obtient :

### EXEMPLE 14 :

**LA 3-{2-**[**4-(4-FLUOROBENZOYL)PIPERIDINO]ETHYL}-6-[2-(4-PYRIDIN-3-YL** **PIPERAZINYL)ETHYL]** **BENZOTHIAZOLIN-2-ONE**
- la 6-{4-[4-(2-méthylphényl)piperazinyl]butyl}benzothiazolin-2-one, on obtient :

### EXEMPLE 15 :

**LA** **3-{2-[4-(4-FLUOROBENZOYL)PIPERIDINO]ETHYL}-6-{4-[4-(2-METHYLPHENYL)****PIPERAZINYL]BUTYL} BENZOTHIAZOLIN-2-ONE**
- la 5-méthoxy-6-(4-morpholinobutyl)benzothiazolin-2-one, on obtient :

### EXEMPLE 16 :

**LA** **3-{2-[4-(4-FLUOROBENZOYL)PIPERIDINO]ETHYL}-5-METHOXY-6-(4-MORPHOLINO****BUTYL) BENZOTHIAZOLIN-2-ONE**
- la 6-[4-(4-naphtylpipérazinyl)butyl]benzothiazolin-2-one, on obtient :

### EXEMPLE 17 :

**LA 3-{2-[4-(4-FLUOROBENZOYL)PIPERIDINO]ETHYL}-6-[4-(4-NAPHTYLPIPERAZINYL)** **BUTYL] BENZOTHIAZOLIN-2-ONE**
- la 6-{4-[4-(4-méthoxynapthyl)pipérazinyl]butyl}benzothiazolin-2-one, on obtient :

### EXEMPLE 18:

**LA 3-{2-[4-(4-FLUOROBENZOYL)PIPERIDINO]ETHYL}-6-{4-[4-(4-METHOXYNAPTHYL)** **PIPERAZINYL] BUTYL} BENZOTHIAZOLIN-2-ONE**
- la 6-{2-[4-(2-méthoxyphényl)pipérazinyl]éthyl}benzothiazolin-2-one, on obtient :

### EXEMPLE 19 :

**LA 3-{2-[4-(4-FLUOROBENZOYL)PIPERIDINO]ETHYL}-6-{2-[4-(2-METHOXYPHENYL)** **PIPERAZINYL]ETHYL} BENZOTHIAZOLIN-2-ONE**
- la 6-[2-(4-phénylpipéridino)éthyl]benzothiazolin-2-one, on obtient :

### EXEMPLE 20 :

**LA** **3-{2-[4-(4-FLUOROBENZOYL)PIPERIDINO]ETHYL}-6-[2-(4-****PHENYLPIPERIDINO)ETHYL]BENZOTHIAZOLIN-2-ONE**
-la 6-[3-(4-phénylpipérazin-1-yl)propyl]benzothiazolin-2-one, on obtient :

### EXEMPLE 21 :

**LA 3-{2-[4-(4-FLUOROBENZOYL)PIPERIDINO]ETHYL}-6-[3-(4-PHENYLPIPERAZINYL)** **PROPYL]BENZOTHIAZOLIN-2-ONE**
- la 6-[3-(4-phénylpipérazin-1-yl)butyl]benzothiazolin-2-one, on obtient :

### EXEMPLE 22 :

**LA 3-{2-[4-(4-FLUOROBENZOYL)PIPERIDINO]ETHYL}-6-[4-(4-PHENYLPIPERAZINYL)** **BUTYL]BENZOTHIAZOLIN-2-ONE**

### EXEMPLE 23 :

**3-{2-[4-(3,4-DIMETHOXYBENZOYL)PIPERIDINO]ETHYL} BENZOTHIAZOLIN-2-ONE**

En procédant comme dans l'exemple 1, mais en partant de la 4-(3,4-diméthoxybenzoyl)pipéridine au lieu de la 4-(4-fluorobenzoyl)pipéridine, on obtient le composé du titre.

### EXEMPLE 24 :

**3-{2-[4-(4-METHOXYBENZOYL)PIPERIDINO]ETHYL} BENZOTHIAZOLIN-2-ONE**

En procédant comme dans l'exemple 1, mais en partant de la 4-(4-méthoxybenzoyl)pipéridine au lieu de la 4-(4-fluorobenzoyl)pipéridine, on obtient le composé du titre.

### EXEMPLE 25 :

**3-[2-(4-BENZOYLPIPERIDINO)ETHYL]BENZOTHIAZOLIN-2-ONE**

En procédant comme dans l'exemple 1, mais en partant de la 4-benzoylpipéridine au lieu de la 4-(4-fluorobenzoyl)pipéridine, on obtient le composé du titre.

### EXEMPLES 26 ET 27 :

En procédant comme dans l'exemple 1, mais en utilisant au lieu de la 4-(4-fluorobenzoyl)pipéridine, les pipéridines convenablement substituées, on obtient les composés des exemples suivants.

### EXEMPLE 26 :

**3-{2-[4-(3,4-DICHLOROBENZOYL)PIPERIDINO]ETHYL} BENZOTHIAZOLIN-2-ONE**

### EXEMPLE 27 :

**3-{2-[4-(4-TRIFLUOROMETHYLBENZOYL)PIPERIDINO]ETHYL} BENZOTHIAZOLIN-2-ONE**

### EXEMPLES 28 A 41 :

En procédant comme dans l'exemple 4, mais en remplaçant la 4-(4-fluorobenzoyl)pipéridine par les pipéridines convenablement substituées, on obtient les composés des exemples suivants :

### EXEMPLE 28 :

**3-{2-[4-(4-METHOXYBENZOYL)PIPERIDINO]ETHYL}-6-(4-MORPHOLINOBUTYL)** **BENZOTHIAZOLIN-2-ONE**

### EXEMPLE 29 :

**3-{2-[4-(4-TRIFLUOROMETHYLBENZOYL)PIPERIDINO]ETHYL}-6-(4-MORPHOLINO-BUTYL)** **BENZOTHIAZOLIN-2-ONE**

### EXEMPLE 30 :

**3-{2-[4-(3,4-DICHLOROBENZOYL)PIPERIDINO]ETHYL}-6-(4-MORPHOLINOBUTYL)** **BENZOTHIAZOLIN-2-ONE**

### EXEMPLE 31 :

**3-[2-(4-BENZOYLPIPERIDINO)ETHYL]-6-(4-MORPHOLINOBUTYL)BENZOTHIAZOLIN-2-ONE**

### EXEMPLE 32 :

**3-{2-[4-(3,5-DIBROMO-2,6-DIMETHOXYBENZOYL)PIPERIDINO]ETHYL}-6-(4-****MORPHOLINOBUTYL)BENZOTHIAZOLIN-2-ONE**

### EXEMPLE 33 :

**3-{2-[4-(4-CHLOROBENZOYL)PIPERIDINO]ETHYL}-6-(4-MORPHOLINOBUTYL)** **BENZOTHIAZOLIN-2-ONE**

### EXEMPLE 34 :

**3-{2-[4-(3-PHENYLPROPIONYL)PIPERIDINO]ETHYL}-6-(4-MORPHOLINOBUTYL)** **BENZOTHIAZOLIN-2-ONE**

### EXEMPLE 35 :

**3-{2-[4-(5-PHENYLVALERYL)PIPERIDINO]ETHYL}-6-(4-MORPHOLINOBUTYL)** **BENZOTHIAZOLIN-2-ONE**

### EXEMPLE 36 :

**3-{2-{4-[4-(4-FLUOROPHENYL)BUTYRYL]PIPERIDINO}ETHYL}-6-(4-MORPHOLINOBUTYL)** **BENZOTHIAZOLIN-2-ONE**

### EXEMPLE 37 :

**3-{3-{[4-(4-FLUOROBENZOYL)]PIPERIDINO}2-METHYLPROPYL}-6-(4-MORPHOLINO****BUTYL) BENZOTHIAZOLIN-2-ONE**

### EXEMPLE 38 :

**3-{2-{4-[(6-FLUORONAPTHYL)CARBONYL]PIPERIDINO}ETHYL}-6-(4-MORPHOLINO****BUTYL) BENZOTHIAZOLIN-2-ONE**

### EXEMPLE 39:

**3-{2-[4-(NAPTHYLCARBONYL)PIPERIDINO]ETHYL}-6-(4-MORPHOLINOBUTYL)** **BENZOTHIAZOLIN-2-ONE**

### EXEMPLE 40 :

**3-{2-{4-[(7-METHOXYNAPHTYL)CARBONYL]PIPERIDINO}ETHYL}-6-(4-MORPHOLINO****BUTYL) BENZOTHIAZOLIN-2-ONE**

### EXEMPLE 41 :

**3-**{**4-[4-(4-FLUOROBENZOYL)PIPERIDINO]BUTYL}-6-(4-MORPHOLINOBUTYL)** **BENZOTHIAZOLIN-2-ONE**

### EXEMPLE 42 :

**3-{2-[4-(4-FLUOROBENZOYL)PIPERIDINO]ETHYL}-6-{3-[4-(2-METHOXYPHENYL)** **PIPERAZINYL]-2-METHYLPROPYL} BENZOTHIAZOLIN-2-ONE**

En procédant comme dans l'exemple 1, mais en remplaçant la benzothiazolin-2-one par la 6-{3-[4-(2-méthoxyphényl)pipérazinyl]-2-méthylpropyl} benzothiazolin-2-one, on obtient le composé du titre.

### EXEMPLE 43 :

**3-{2-[4-(4-FLUOROBENZOYL)PIPERIDINO]ETHYL**} **BENZOXAZOLIN-2-ONE**

En procédant comme dans l'exemple 1, mais en remplaçant la benzothiazolin-2-one par la benzoxazolin-2-one, on obtient le composé du titre.

### EXEMPLE 44 :

**3-{2-[4-(4-FLUOROBENZOYL)PIPERIDINO]ETHYL}-6-{4-[4-(3-TRIFLUOROMETHYL-** **PHENYL)PIPERAZINYL]BUTYL} BENZOXAZOLIN-2-ONE**

En procédant comme dans l'exemple 1, mais en remplaçant la 6-{4-[4-(3-trifluorométhylphényl)pipérazinyl]butyl}benzothiazolin-2-one par la 6-{4-[4-(3-trifluorométhylphényl)pipérazinyl]butyl}benzoxazolin-2-one, on obtient le composé du titre.

### EXEMPLE 45 :

**3-{2-[4-(4-FLUOROBENZOYL)PIPERIDINO]ETHYL}-6-{4-[4-(2-METHOXYPHENYL)** **PIPERAZINYL]BUTYL} BENZOXAZOLIN-2-ONE**

En procédant comme dans l'exemple 3, mais en remplaçant la 6-{4-[4-(2-méthoxyphényl)pipérazin-1-yl]butyl}benzothiazolin-2-one par la 6-{4-[4-(2-méthoxyphényl)pipérazin-1-yl]butyl}benzoxazolin-2-one, on obtient le composé du titre.

### EXEMPLE 46 :

**3-{2-[4-(4-FLUOROBENZOYL)PIPERIDINO]ETHYL}-6-(4-MORPHOLINOBUTYL)** **BENZOXAZOLIN-2-ONE**

En procédant comme dans l'exemple 4, mais en remplaçant la 6-(4-morpholinobutyl)benzothiazolin-2-one par la 6-(4-morpholinobutyl) benzoxazolin-2-one, on obtient le composé du titre.

### EXEMPLE 47 :

**LA** **3-{2-[4-(4-FLUOROBENZOYL)PIPERAZINYL]ETHYL}BENZOTHIAZOLIN-2-ONE**

En procédant comme dans l'exemple 1, mais en partant, au stade A, de l'acide pipérazinecarboxilyque-1 au lieu de l'acide pipéridinecarboxylique-4, on obtient le composé du titre.

### EXEMPLE 48 :

**3-METHYL-6-{2-[4-(4-FLUOROBENZOYL)PIPERIDINO]ETHYL}BENZOTHIAZOLIN-2-ONE**

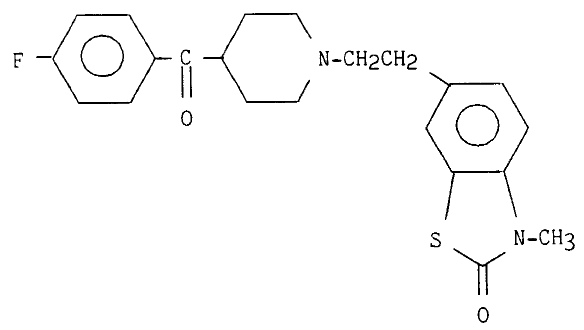

### STADE A :

**3-METHYL-6-(2-BROMOETHYL)BENZOTHIAZOLIN-2-ONE**

Dans une fiole à col rodé de 250 cm³, dissoudre 0,15 mole de 3-méthyl-6-bromoacétylbenzothiazolin-2-one (42,9 g) dans 1 mole d'acide trifluoroacétique (77 cm³). A l'aide d'une ampoule à brome, introduire goutte à goutte et sous agitation magnétique 0,33 mole de triéthylsilane (52,70 cm³). Adapter une garde de chlorure de calcium et maintenir l'agitation pendant le temps nécessaire à température ambiante. Verser le mélange réactionnel dans 500 cm³ d'eau glacée. Essorer le précipité obtenu, le laver à l'eau jusqu'à neutralité des eaux de lavage, le sécher puis le recristalliser.
Temps de réaction : 20 heures
Point de fusion : 97-98°C
Rendement : 86%
Solvant de recristallisation : cyclohexane

| Analyse élémentaire : | | | |
|---|---|---|---|
| Calculée : | C% 44,13 | H% 3,70 | N% 5,15 |
| Trouvée : | C% 44,26 | H% 3,60 | N% 5,34 |

| Spectrométrie dans l'Infrarouge : | |
|---|---|
| 3050-2850 cm⁻¹ | ν CH (alkyles) |
| 1660 cm⁻¹ | ν CO (NCOS) |
| 1610-1580 cm⁻¹ | ν C=C (aromatiques) |

### STADE B

**3-METHYL-6-{2-[4-(4-FLUOROBENZOYL)PIPERIDINO]ETHYL}BENZOTHIAZOLIN-2-ONE**

Dissoudre 0,01 mole de la 3-méthyl-6-(2-bromoéthyl)benzothiazolin-2-one dans 50 cm³ d'acétone anhydre. Chauffer à reflux de l'acétone.
Ajouter 0,022 mole de triéthylamine en solution dans 20 cm³ d'acétone anhydre puis, sous agitation magnétique, 0,01 mole de chlorhydrate de la 4-(4-fluorobenzoyl)pipéridine. Continuer le chauffage pendant 24 heures puis essorer le bromhydrate de triéthylamine formé.
Evaporer le filtrat et reprendre le résidu par 50 cm³ d'une solution de HCl 1M. Le produit attendu précipite, l'essorer, le sécher puis le recristalliser.
Point de fusion (chlorhydrate) : 224-226°C
Rendement : 62%
Solvant de recristallisation : alcool absolu

| Dosage d'azote basique : pour un azote basique | |
|---|---|
| Pourcentage d'azote basique théorique : | 3,22% |
| Pourcentage d'azote basique trouvé : | 2,94% |

| Spectrométrie dans l'Infrarouge : | |
|---|---|
| 3000-2800 cm⁻¹ | ν CH (alkyles) |
| 2600-2400 cm⁻¹ | ν NH |
| 1660 cm⁻¹ | ν CO (NCOS) |
| 1660 cm⁻¹ | ν CO (cétone) |
| 1600-1580 cm⁻¹ | ν C=C (aromatiques) |

### EXEMPLE 49 :

**3-METHYL-6-{4-[4-(4-FLUOROBENZOYL)PIPERIDINO]BUTYL}BENZOTHIAZOLIN-2-ONE**

En procédant comme dans l'exemple 48, mais en remplaçant au stade A la 3-méthyl-6-bromoacétylbenzothiazolin-2-one par la 3-méthyl-6-bromobutyrylbenzothiazolin-2-one, on obtient le produit du titre.

### EXEMPLES 50 à 52 :

En procédant comme dans l'exemple 48, mais en remplaçant au stade B la 4-(4-fluorobenzoyl)pipéridine par les pipéridines convenablement substituées, on obtient les composés des exemples suivants :

### EXEMPLE 50 :

**LA 3-METHYL-6-{4-[4-(4-CHLOROBENZOYL)PIPERIDINO]ETHYL}BENZOTHIAZOLIN-2-ONE**

### EXEMPLE 51 :

**LA 3-METHYL-6-[4-(4-BENZOYLPIPERIDINO]ETHYL]BENZOTHIAZOLIN-2-ONE**

### EXEMPLE 52 :

**LA** **3-METHYL-6-{4-[4-(4-METHOXYBENZOYL)PIPERIDINO]ETHYL}BENZOTHIAZOLIN-2-****ONE**

### EXEMPLE 53 :

**LA 3-METHYL-6-**{**2-[4-(4-METHOXYBENZOYL)PIPERIDINO]ETHYL}BENZOXAZOLIN-2-ONE**

En procédant comme dans l'exemple 48, mais en remplaçant au stade A la 3-méthyl-6-bromoacétylbenzothiazolin-2-one par la 3-méthyl-6-bromoacétylbenzoxazolin-2-one, on obtient le produit du titre.

### EXEMPLE 54 :

**LA 6-{2-[4-(4-FLUOROBENZOYL)PIPERIDINO]ETHYL}BENZOTHIAZOLIN-2-ONE**

En procédant comme dans l'exemple 48, mais en remplaçant la 3-méthyl-6-bromoacétylbenzothiazolin-2-one par la 6-bromoacétylbenzothiazolin-2-one, on obtient le composé du titre.

### EXEMPLE 55 :

**LA 6-{4-[4-(4-FLUOROBENZOYL)PIPERIDINO]BUTYL}BENZOTHIAZOLIN-2-ONE**

En procédant comme dans l'exemple 48, mais en remplaçant au stade A la 3-méthyl-6-bromoacétylbenzothiazolin-2-one par la 6-bromobutyrylbenzothiazolin-2-one, on obtient le produit du titre.

### EXEMPLES 56 à 58 :

En procédant comme dans l'exemple 55, mais en remplaçant au stade B la 4-(4-fluorobenzoyl)pipéridine par les pipéridines convenablement substituées, on obtient les composés des exemples suivants :

### EXEMPLE 56 :

**LA 6-**{**4-[4-(4-CHLOROBENZOYL)PIPERIDINO]BUTYL}BENZOTHIAZOLIN-2-ONE**

### EXEMPLE 57 :

**LA 6-[4-(4-BENZOYLPIPERIDINO)BUTYL]BENZOTHIAZOLIN-2-ONE**

### EXEMPLE 58 :

**LA 6-**{**4-[4-(4-METHOXYBENZOYL)PIPERIDINO]BUTYL}BENZOTHIAZOLIN-2-ONE**

### EXEMPLE 59 :

**LA 3-METHYL-6-{4-[4-(4-FLUOROBENZOYL)PIPERIDINO]BUTYL}BENZOXAZOLIN-2-ONE**

En procédant comme dans l'exemple 48, mais en remplaçant la 3-méthyl-6-bromoacétylbenzothiazolin-2-one par la 3-méthyl-6-bromobutyrylbenzoxazolin-2-one, on obtient le produit du titre.

### EXEMPLES 60 à 62 :

En procédant comme dans l'exemple 59, mais en remplaçant au stade B la 4-(4-fluorobenzoyl)pipéridine par les pipéridines convenablement substituées, on obtient les composés des exemples suivants :

### EXEMPLE 60 :

**LA 3-METHYL-6-{4-[4-(4-CHLOROBENZOYL)PIPERIDINO]BUTYL}BENZOXAZOLIN-2-ONE**

### EXEMPLE 61 :

**LA 3-METHYL-6-[4-(4-BENZOYLPIPERIDINO)BUTYL]BENZOXAZOLIN-2-ONE**

### EXEMPLE 62 :

**LA 3-METHYL-6-{4-[4-(4-METHOXYBENZOYL)PIPERIDINO]BUTYL}BENZOXAZOLIN-2-ONE**

### EXEMPLE 63 :

**LA 6-{4**-**[4-(4-FLUOROBENZOYL)PIPERIDINO]BUTYL}BENZOXAZOLIN-2-ONE**

En procédant comme dans l'ensemble 48, mais en remplaçant la 3-méthyl-6-bromoacétylbenzothiazolin-2-one par la 6-bromobutyrylbenzoxazolin-2-one, on obtient le produit du titre.

### EXEMPLES 64 à 66 :

En procédant comme dans l'exemple 63, mais en remplaçant au stade B la 4-(4-fluorobenzoyl)pipéridine par les pipéridines convenablement substituées, on obtient les composés des exemples suivants :

### EXEMPLE 64 :

**LA 6-{4-[4-(4-CHLOROBENZOYL)PIPERIDINO]BUTYL}BENZOXAZOLIN-2-ONE**

### EXEMPLE 65 :

**LA 6-[4-(4-BENZOYLPIPERIDINO)BUTYL]BENZOXAZOLIN-2-ONE**

### EXEMPLE 66 :

**LA 6-{4-[4-(4-METHOXYBENZOYL)PIPERIDINYL]BUTYL}BENZOXAZOLIN-2-ONE**

### EXEMPLE A :

**MESURE DE L'AFFINITE POUR LES RECEPTEURS SEROTONINERGIQUES**

### PROTOCOLE :

L'affinité in-vitro des composés de l'invention a été déterminée :
- pour les récepteurs sérotoninergiques 5HT_{1A}, par la mesure du déplacement du 8-OH-DPAT (8-hydroxy-2-(di-n-propylamino)tétraline), sur des préparations d'hippocampes de rat,
- pour les récepteurs sérotoninergiques 5HT_{1C}, par la mesure du déplacement du N-méthylmésulergine, sur des préparations de cortex frontal et d'hippocampe de rat,
- pour les récepteurs sérotoninergiques 5HT_{1D}, par la mesure du déplacement de la 5-hydroxy-tryptamine, sur des préparations de cortex, striatum et globus pallidus de rat,
- pour les récepteurs sérotoninergiques 5HT₂, par la mesure du déplacement de l'amino-idodo-kétansérine, sur des préparations de cortex frontal de rat,
- pour les récepteurs sérotoninergiques 5HT₃, par la mesure du déplacement du BRL 43694, sur des préparations d'aéra postréma de rat.

### RESULTATS :

Les composés de l'invention montrent une très forte affinité pour les récepteurs sérotoninergiques 5-HT2. A titre d'exemples, les composés des exemples 1 et 3 possèdent une IC₅₀ (concentration inhibant de 50% la liaison du ligand marqué) de l'ordre de 10⁻⁹ M à 10⁻¹⁰ M (composés des exemples 1 et 3 : IC₅₀ : 10⁻⁹ M, composé de l'exemple 4 : 5.10⁻¹⁰ M).

Les composés de l'invention montrent également une importante sélectivité de liaison aux récepteurs 5HT-2 par rapport aux autres récepteurs sérotoninergiques.

Les composés de la demande EP 506539 ne présentent aucunement une telle affinité et sélectivité pour les récepteurs 5-HT₂.

### EXEMPLE B :

**ACTIVITE ANTI-PSYCHOTIQUE : ETUDE DE L'ANTAGONISME DE L'HYPERACTIVITE** **INDUITE PAR L'AMPHETAMINE**

L'antagonisme sélectif de l'hyperactivité induite par l'amphétamine est considéré comme indicateur d'une activité antipsychotique.

### PROTOCOLE :

Une injection par voie IP de 2 mg/kg d'amphétamine induit une hyperactivité marquée mesurable. Des rats Sprague-Dawley pesant 200 à 250 g reçoivent les composés à tester par voie IP avant l'administration de l'amphétamine et l'activité locomotrice est alors mesurée 30 minutes plus tard pour une période de 30 minutes. 12 animaux sont testés par dose.
Référence : Costall B. et al. - Brain Res. 123 : 89-111.

### RESULTATS :

Les composés de l'invention inhibent de façon très importante l'hyperactivité induite par l'amphétamine.

### EXEMPLE C :

**MESURE DE L'ACTIVITE ANXIOLYTIQUE : TEST DES CAGES CLAIRES-OBSCURES**

Les rats préfèrent les espaces clos et sombres aux espaces ouverts et éclairés. Cette préférence se traduit par la proportion du temps passé dans les espaces clos et sombres. Une caractéristique des composés anxiolytiques est d'augmenter le temps passé dans les espaces ouverts et éclairés.

### PROTOCOLE :

Les animaux sont placés dans une cage constituée de 2 compartiments : l'un étant ouvert et éclairé, l'autre étant sombre et clos. Le temps passé par l'animal dans chacun des compartiments ainsi que le nombre de passages d'un compartiment à l'autre est mesuré durant une période de 5 minutes. 10 animaux sont étudiés par dose.
Référence : Crawley J.N., Pharmacol. Biochem. Behav. 1981, vol 15, p 695-699.

### RESULTATS :

Il apparaît clairement que les composés de l'invention ont une activité anxiolytique puisqu'ils augmentent de façon très significative le temps passé par les animaux dans le compartiment éclairé.

### EXEMPLE D :

**ACTIVITE ANALGESIQUE : TEST DE LA PLAQUE CHAUFFANTE**

On place des rats ou des souris sur une plaque chaude (58°C) à l'intérieur d'un cylindre en plexiglas®. On mesure le temps de latence que met l'animal pour lécher ses pattes. Si aucune réaction n'est notée, on termine le test après 120 secondes. On étudie 10 animaux par dose. Le composé testé est habituellement administré i.p. 30 minutes avant le test.

On utilise 16 mg.kg.⁻¹ i.p. de morphine comme composé de référence qui inhibe de 129 % le temps de latence que met l'animal pour lécher ses pattes.

Référence : Eddy N.B., Leimbach D., 1959. Synthetic analgesics : II-dithiénylbutenyl and dithienylbutylamines. J.Pharmacol. Exp. Ther., 107 : 385-393.

Résultats : les composés de l'invention augmente de façon très importante le temps de latence que met l'animal pour lécher ses pattes. Par exemple, 0,25 mg.kg⁻¹ du composé de l'exemple 48 inhibé de 175 % ce temps de latence.

### EXEMPLE E :

**MESURE DE LA TOXICITE**

La toxicité a été testée après administration orale d'une dose de 650 mg/kg à des groupes de 8 souris (26 ± 2 grammes). Les animaux sont observés à intervalles réguliers durant le premier jour et quotidiennement durant les 2 semaines suivant le traitement.

Il apparaît que les composés de l'invention ne sont pas toxiques à une dose de 650 mg/kg, et aucun trouble n'est en général observé après l'administration d'une telle dose.

### EXEMPLE F :

**COMPOSITION PHARMACEUTIQUE**

Comprimé dosé à 2,5 mg de 3-{2-[4-(4-fluorobenzoyl)piperidino]éthyl}-6-(4-morpholinobutyl)benzothiazolin-2-one

| Préparation pour 1000 comprimés : | |
|---|---|
| 3-{2-[4-(4-fluorobenzoyl)piperidino]éthyl}-6-(4-morpholinobutyl) benzothiazolin-2-one | 2,5 g |
| Amidon de blé | 15 g |
| Amidon de maïs | 15 g |
| Lactose | 65 g |
| Stéarate de magnésium | 2 g |
| Silice | 1 g |
| Hydroxypropylcellulose | 2 g |

## Revendications

1. Composés de formule (I) : 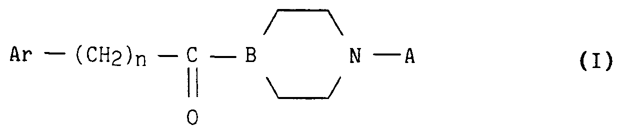 dans laquelle :
Ar : représente un groupement phényle ou naphtyle, Ar étant non substitué ou substitué par un ou plusieurs radicaux choisis parmi halogène, alkyle inférieur, alkoxy inférieur et trifluorométhyle,
n : représente 0 ou un nombre entier de 1 à 4,
B : représente un groupement -CH〈 ou -N〈 , et
A : représente un groupement de formule (A1) ou (A2) : 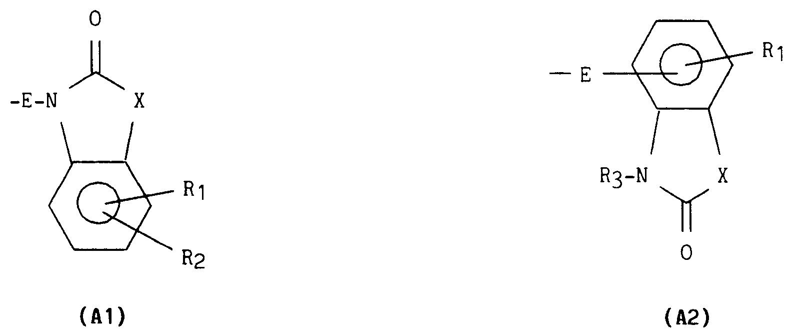
dans lesquelles :
E : représente une chaîne alkylène linéaire ou ramifiée de 1 à 6 atomes de carbone,
R₁ : représente un radical choisi parmi hydrogène, hydroxy, alkyle inférieur et alkoxy inférieur,
R₂ : représente un radical choisi parmi hydrogène, alkyle inférieur, et 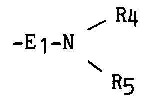 où E₁ a la même définition que E tel que décrit précédemment et où R₄ et R₅ sont choisis indépendamment l'un de l'autre parmi hydrogène et alkyle inférieur, ou forment ensemble, avec l'atome d'azote qui les porte, un hétérocycle choisi parmi pyrrolidine, pipéridine, pipéridine substituée, morpholine, pipérazine, et pipérazine substituée,
R₃ : représente un radical choisi parmi hydrogène et alkyle inférieur, et
X : représente un atome de soufre ou d'oxygène,
leurs isomères optiques,
et leurs sels d'addition à une base ou a un acide pharmaceutiquement acceptable,
étant entendu que, sauf précision contraire, les termes "alkyle inférieur" et "alcoxy inférieur" désignent des groupements linéaires ou ramifiés de 1 à 6 atomes de carbone, et le terme "substitué" affecté aux hétérocycles "pipéridine" et "pipérazine" signifie que ces hétérocycles peuvent être substitués en position 4 par un radical choisi parmi alkyle inférieur, aryle et arylalkyle inférieur ; le terme "aryle" désignant un groupement phényle, naphtyle ou pyridinyle, et pouvant lui-même être non substitué ou substitué par un ou plusieurs radicaux choisis parmi halogène, alkyle inférieur, hydroxyle, alkoxy inférieur et trifluorométhyle.

2. Composés selon la revendication 1, pour lesquels Ar représente un groupement 4-fluorophényle,
leurs isomères optiques,
et leurs sels d'addition à une base ou à un acide pharmaceutiquement acceptable.

3. Composés selon la revendication 1, pour lesquels Ar représente un groupement 4-fluorophényle, n est égal à zéro et B représente un groupement -CH〈 ,
leurs isomères optiques,
et leurs sels d'addition à une base ou à un acide pharmaceutiquement acceptable.

4. Composés selon la revendication 1, pour lesquels R₂ représente un groupement 4-morpholinobutyle,
leurs isomères optiques,
et leurs sels d'addition à une base ou à un acide pharmaceutiquement acceptable.

5. Composé selon la revendication 1, qui est la 3-{2-[4-(4-fluorobenzoyl)pipéridino]éthyl}benzothiazolin-2-one et ses sels d'addition à un acide pharmaceutiquement acceptable.

6. Composé selon la revendication 1, qui est la 3-{2-[4-(4-fluorobenzoyl)pipéridino]éthyl}-6-{4-[4-(3-trifluorométhylphényl) pipéra- zinyl]butyl}benzothiazolin-2-one et ses sels d'addition à un acide pharmaceutiquement acceptable.

7. Composé selon la revendication 1, qui est la 3-{2-[4-(4- fluorobenzoyl)pipéridino]éthyl}-6-(4-morpholinobutyl)benzothiazolin-2-one et ses sels d'addition à un acide pharmaceutiquement acceptable.

8. Composé selon la revendication 1, qui est la 3-méthyl 6-{4-[4-(4- fluorobenzoyl)pipéridinyl]butyl}benzothiazolin-2-one et ses sels d'addition à un acide pharmaceutiquement acceptable.

9. Procédé d'obtention des composés de formule (I) selon la revendication 1 caractérisé en ce que l'on fait réagir un composé de formule (II) : 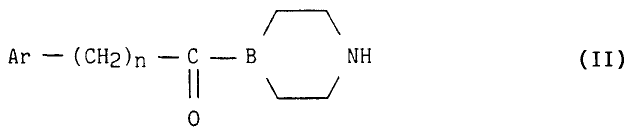 dans laquelle Ar, n et B sont tels que définis dans la formule (I) selon la revendication 1,
avec un composé de formule (III/A1) : 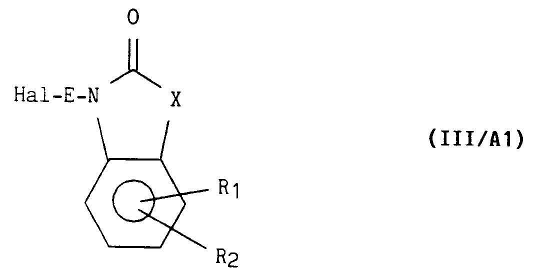 ou de formule (III/A2) : 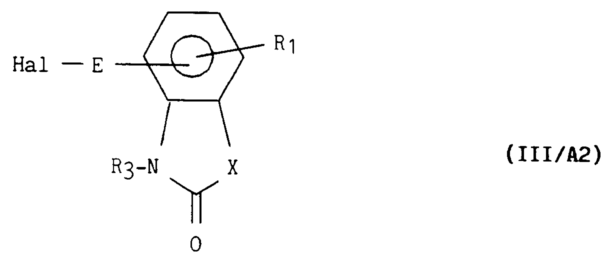 dans lesquelles E, X, R₁, R₂, et R₃ sont tels que définis dans la formule (I) selon la revendication 1, et Hal représente un atome d'halogène, pour obtenir le composé de formule (I) selon la revendication 1 correspondant,
les composés de formule (I) étant le cas échéant :
- purifiés suivant une ou plusieurs méthodes de purification choisies parmi la cristallisation, la chromatographie sur colonne de silice, l'extraction, la filtration, et le passage sur charbon ou résine,
- séparés, sous forme pure ou sous forme de mélange, en leurs isomères optiques,
- et salifiés par un acide ou une base pharmaceutiquement acceptable.

10. Procédé d'obtention des composés de formule (I/a) : 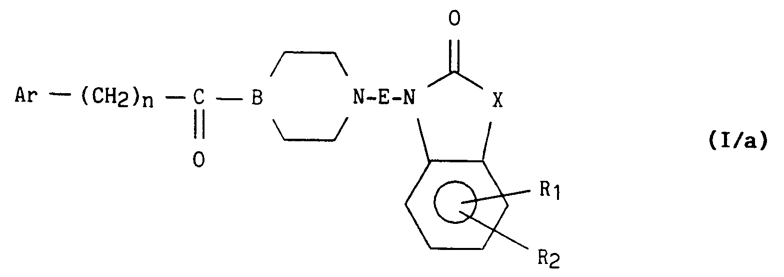 dans laquelle Ar, n, B, E, X, R₁ et R₂ sont tels que définis dans la formule (I) selon la revendication 1,
caractérisé en ce que l'on fait réagir un composé de formule (IV) : 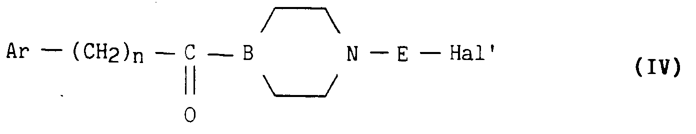 dans laquelle Ar, n, B et E sont tels que définis précédemment et Hal' représente un atome d'halogène, avec un composé de formule (V) : 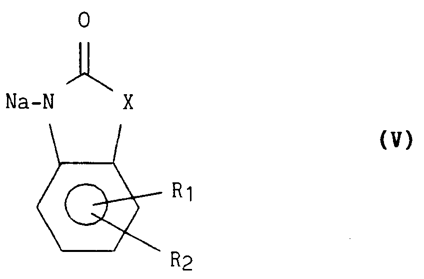 dans laquelle X, R₁ et R₂ sont tels que décrits précédemment, afin d'obtenir les composés de formule (I/a) correspondants,
les composés de formule (I/a) étant le cas échéant :
- purifiés suivant une ou plusieurs méthodes de purification choisies parmi la cristallisation, la chromatographie sur colonne de silice, l'extraction, la filtration, et le passage sur charbon ou résine,
- séparés, sous forme pure ou sous forme de mélange, en leurs isomères optiques,
- et salifiés par un acide ou une base pharmaceutiquement acceptable.

11. Compositions pharmaceutiquse contenant comme principe actif un ou plusieurs composés de formule (I) selon la revendication 1, ou leurs sels d'addition à un acide ou à une base pharmaceutiquement acceptable, en combinaison avec un ou plusieurs excipients, véhicules ou diluants pharmaceutiquement acceptables.

12. Composition pharmaceutique selon la revendication 11 utile dans le traitement et la prévention des pathologies nécessitant des agents psychotropiques.

13. Composition pharmaceutique selon la revendication 12 utile dans le traitement et la prévention de l'anxiété, de la dépression et des syndrômes dépressifs, des états psychotiques et de la maladie de Parkinson.

14. Composition pharmaceutique selon la revendication 11 utile pour le traitement de la douleur.
